Europäisches Patentamt

**European Patent Office**

Office européen des brevets

(19)

(11) Veröffentlichungsnummer : **0 092 170 B1**

(12) **EUROPÄISCHE PATENTSCHRIFT**

(45) Veröffentlichungstag der Patentschrift :
12.11.86

(51) Int. Cl.⁴ : **A 61 B 17/30**

(21) Anmeldenummer : **83103600.9**

(22) Anmeldetag : **14.04.83**

(54) **Bipolare Pinzette.**

(30) Priorität : **19.04.82 DE 3214318**

(43) Veröffentlichungstag der Anmeldung :
**26.10.83 Patentblatt 83/43**

(45) Bekanntmachung des Hinweises auf die Patenterteilung : **12.11.86 Patentblatt 86/46**

(84) Benannte Vertragsstaaten :
**AT BE CH DE FR GB IT LI LU NL SE**

(56) Entgegenhaltungen :
**DE-A- 3 012 849**
**US-A- 3 911 241**
**L.I. MAISSEL u.a.: "Handbook of thin film technology,**
**1970, Seiten 19-20, McGraw-Hill, New York, US**

(73) Patentinhaber : **S + T Marketing AG**
**Zollstrasse 91**
**CH-8212 Neuhausen am Rheinfall (CH)**

(72) Erfinder : **Tritt, Eugen**
**Hegauweg 11**
**D-7893 Jestetten (DE)**

(74) Vertreter : **Fiedler, Otto Karl, Dipl.-Ing.**
**Patentanwalt Hug Interlizenz AG Austrasse 44 Post-**
**fach**
**CH-8045 Zürich (CH)**

## Beschreibung

Die Erfindung bezieht sich auf eine bipolare Pinzette mit zwei metallenen Armen, die am einen Ende mechanisch miteinander verbunden, jedoch elektrisch voneinander isoliert sind. Eine derartige Pinzette ist aus der Druckschrift DE-A-3 012 849 bekannt.

Solche Pinzetten können insbesondere in der Mikrochirurgie als sogenannte Koagulationspinzetten verwendet werden, mit denen mit Hilfe einer an die beiden Arme angelegten elektrischen Spannung (Hochfrequenzspannung) kleine Blutgefässe verödet werden können.

Bei bekannten Koagulationspinzetten sind die hinteren Endabschnitte der beiden Arme in einem Kunststoffkörper festgehalten, der die Arme mechanisch miteinander verbindet und elektrisch voneinander isoliert. Der Kunststoff ist jedoch nur begrenzt hitzebeständig, so dass beim notwendigen Sterilisieren dieser Pinzetten die Temperaturen beschränkt bleiben müssen. Trotzdem werden Lebensdauer und Festigkeit des Kunststoffs durch die Sterilisationen beeinträchtigt.

Die Aufgabe der Erfindung besteht darin, diese Nachteile zu vermeiden und eine bipolare Pinzette zur Verfügung zu stellen, die auch sehr hohe Temperaturen ohne Schaden aushält.

Die Aufgabe ist bei der erfindungsgemässen Pinzette dadurch gelöst, dass die hinteren Endabschnitte der Arme durch einen Metallhalter festgehalten sind, der wenigstens mit einem dieser hinteren Endabschnitte zumindest über einen auf demselben durch Plasmabeschichtung aufgebrachten Oxidüberzug in Berührung steht.

Die Plasmabeschichtung ist ein bekanntes Verfahren zum Erzeugen von Ueberzügen aus Oxiden, in der Regel hauptsächlich Aluminiumoxid, auf Metallteilen. Die Plasmabeschichtung wird z. B. in der Flugzeugindustrie angewandt, um Metallteile mit einer harten, abriebfesten Oberflächenschicht zu versehen.

Bei der erfindungsgemässen Pinzette, die vor allem die elektrische Isolationswirkung und die Hitzebeständigkeit dieser Oxidüberzüge ausnutzt, kann die Härte und Abriebfestigkeit zusätzliche Vorteile bieten.

Ein Ausführungsbeispiel der erfindungsgemässen bipolaren Pinzette ist in der Zeichnung schematisch dargestellt, und zwar zeigen

Figur 1 eine perspektivische Ansicht der Pinzette und

Figur 2 ein Querschnitt durch den die beiden Arme der Pinzette miteinander verbindenden, aus Metall bestehenden Halter.

Die dargestellte Pinzette besitzt zwei federnde Arme 1 und 2 von üblicher Form aus rostfreiem Stahl.

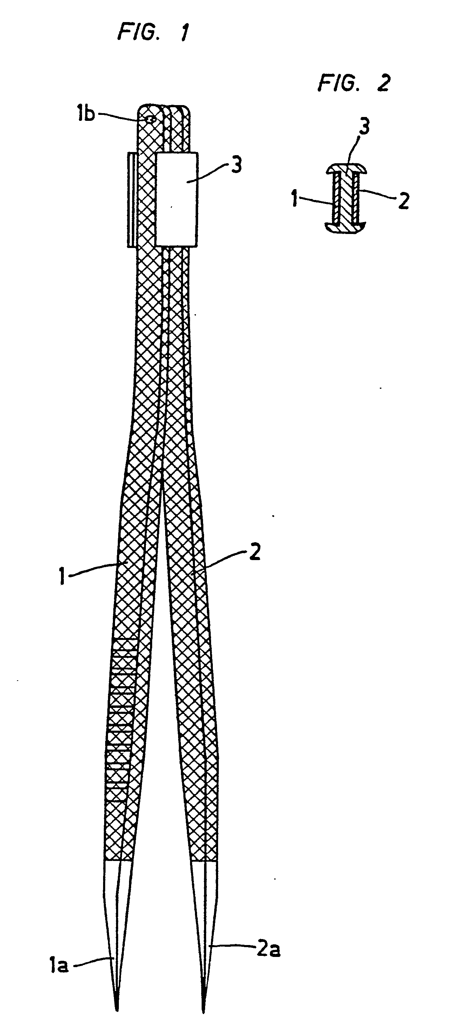

Die Arme 1 und 2 sind auf dem grössten Teil ihrer hinter den Spitzenabschnitten 1a bzw. 2a liegenden Länge, wie in Figur 1 durch Kreuzschraffierung angedeutet, mit einem durch Plasmabeschichtung aufgebrachten Oxidüberzug umhüllt. Bei der Plasmabeschichtung werden geschmolzene Oxidteilchen, hauptsächlich Aluminiumoxid, auf die Oberfläche der Arme 1 und 2 geschleudert.

Die hinteren Endabschnitte der beiden Arme 1 und 2 sind in einem Halter 3 festgehalten, der aus Metall besteht, vorzugsweise aus rostfreiem Stahl. Da der Halter 3 nur den elektrisch nicht leitenden Oxidüberzug der Arme 1 und 2 berührt, sind die Arme vom Halter und voneinander elektrisch isoliert. Zwischen den Armen 1 und 2 kann daher über geeignete Anschlüsse eine elektrische Spannung angelegt (oder abgenommen) werden. In Fig. 1 ist als Anschluss eine nach dem Beschichten angebrachte Senkbohrung 1b dargestellt, in der das Metall des Armes 1 zutage tritt.

Wenn man an die Arme 1 und 2 eine geeignete Hochfrequenzspannung legt, kann die Pinzette als Koagulationspinzette in der Mikrochirurgie zum Veröden von kleinen Blutgefässen verwendet werden. Andere Verwendungsmöglichkeiten der bipolaren Pinzette, beispielsweise in der Elektronikindustrie, sind jedoch ebenfalls denkbar.

Dank der Beschichtung der beiden Arme 1 und 2 mit den Oxidüberzügen und deren mechanischer Verbindung mittels der Metallklammer 3 besitzt die beschriebene Pinzette, im Vergleich zu den bekannten bipolaren Pinzetten mit Kunststoffhalter, insbesondere die folgenden Vorteile : Höhere Widerstandsfähigkeit gegen mechanische Beanspruchung, Säurebeständigkeit und wesentlich bessere Widerstandsfähigkeit gegen die Beanspruchung bei der Sterilisation im Dampfautoklaven. Der Oxidüberzug ist hitzebeständig bis etwa 2 000 °C und weist einen hohen Isolationswert auf.

In der bevorzugten Ausführungsform sind die Arme der bipolaren Pinzette wie dargestellt mit Ausnahme der vorderen Spitzenabschnitte 1a, 2a und der hinteren Anschlussstellen 1b vollständig mit den Oxidüberzügen umhüllt, also auf etwa 4/5 ihrer Länge. Da es aber zur Hauptsache darauf ankommt, die Arme gegenüber der Metallklammer 3 elektrisch zu isolieren, würde es natürlich im Prinzip ausreichen, nur die mit der Metallklammer in Berührung stehenden Oberflächen der hinteren Endabschnitte der Arme mit Oxidüberzügen zu beschichten. Ferner ist auch denkbar, nur einen der beiden Arme mit einem Oxidüberzug zu versehen, da das schon ausreicht, um die beiden Arme elektrisch voneinander zu isolieren.

Um eine Beschädigung der Oxidschicht beim Aufpressen der Metallklammer 3 zu vermeiden, kann es vorteilhaft sein, zwischen dieser und den Enden der Arme 1 und 2 eine Druckausgleichsschicht (nicht dargestellt) vorzusehen. Damit wird die Druckbeanspruchung auf die makroskopisch gesehen unebene Oberfläche des Oxidüberzugs vergleichmässigt und die Gefahr der örtlichen Beeinträchtigung der Isoliereigenschaft des Oxidüberzugs ausgeschaltet. Die Druckausgleichsschicht braucht nicht unbedingt elektrisch isolierend, soll aber vorzugsweise thermisch belastbar und alterungsbeständig sein.

**Patentansprüche**

1. Bipolare Pinzette, mit zwei metallenen Armen (1 und 2), die am einen Ende mechanisch miteinander verbunden, jedoch elektrisch voneinander isoliert sind, dadurch gekennzeichnet, dass die hinteren Endabschnitte der Arme (1 und 2) durch einen Metallhalter (3) festgehalten sind, der wenigstens mit einem dieser hinteren Endabschnitte zumindest über einen auf demselben durch Plasmabeschichtung aufgebrachten, elektrisch isolierenden und hitzebeständigen Oxidüberzug in Berührung steht.

2. Bipolare Pinzette nach Anspruch 1, dadurch gekennzeichnet, dass beide Arme (1 und 2) auf einem grösseren Teil ihrer Länge, z. B. etwa 4/5 ihrer Länge, von den Oxidüberzügen umhüllt sind.

3. Bipolare Pinzette nach Anspruch 1 oder 2, dadurch gekennzeichnet, dass die Oxidüberzüge überwiegend aus Aluminiumoxid bestehen.

4. Bipolare Pinzette nach einem der Ansprüche 1 bis 3, dadurch gekennzeichnet, dass zwischen Oxidüberzug und Metallhalter (3) eine Druckausgleichsschicht vorgesehen ist.

**Claims**

1. Bipolar forceps comprising two metallic arms (1 and 2) which at one end are mechanically connected to each other, but electrically insulated from each other, characterized in that the rear end sections of said arms (1 and 2) are retained by a metal holder (3) being in contact with at least one of said rear end sections over at least one electrically insulating and heat-resisting oxide layer applied thereon by plasma coating.

2. Bipolar forceps according to claim 1, characterized in that both arms (1 and 2) are covered by oxide layers on a greater part, e. g. about 4/5, of their length.

3. Bipolar forceps according to claim 1 or 2, characterized in that said oxide layers are predominantly composed of aluminium oxide.

4. Bipolar forceps according to one of the claims 1 to 3, characterized in that, between oxide layer and metal holder (3), there is provided for a pressure equalizing layer.

**Revendications**

1. Pince bipolaire possédant deux bras métalliques (1 et 2) qui sont liés mécaniquement entre eux à l'une de leurs extrémités, mais électriquement isolés l'un de l'autre, caractérisée en ce que les sections finales arrières des bras (1 et 2) sont retenues par un support métallique (3) qui est mis en contact avec au moins l'une de ces sections finales arrières par l'intermédiaire d'au moins une couche d'oxyde appliquée sur cette partie du bras par enduction plasmatique, cette couche d'oxyde étant électriquement isolante et résistante à la chaleur.

2. Pince bipolaire selon la revendication 1, caractérisée en ce que les deux bras (1 et 2) sont enrobés par des couches d'oxyde sur une grande partie, par exemple sur 4/5, de leur longueur.

3. Pince bipolaire selon la revendication 1 ou 2, caractérisée en ce que les couches d'oxyde consistent d'une manière prépondérante en oxyde d'aluminium.

4. Pince bipolaire selon l'une des revendications 1 à 3, caractérisée en ce qu'il est prévu une couche d'équilibre de pression entre la couche d'oxyde et le support métallique (3).

FIG. 1

FIG. 2